# DEMANDE DE BREVET EUROPEEN

(11) **EP 2 002 794 A1**
(43) Date de publication de la demande: **17.12.2008**
(21) Numéro de dépôt: 08158099.5
(22) Date de dépôt: 12.06.2008
(51) Int. Cl.: A61B 17/17, A61F 2/40

(54) **Ensemble comprenant une prothèse d'épaule et un instrument de pose de cette prothèse d'épaule**

(30) Priorité: 13.06.2007 FR 0704213
(71) Demandeur: Compagnie Financière et Médicale, 01000 Bourg en Bresse (FR)
(72) Inventeur: Martin, Jean-Jacques, 01000 Bourg en Bresse (FR); Lascar, Tristan, 06320 Cap D'ail (FR)
(74) Mandataire: Jeannet, Olivier

(57) **Abrégé**

La prothèse d'épaule inclut un élément huméral (1) qui comprend une tige effilée (3), cette tige effilée (3) comprenant au moins un trou à vis (4), et l'instrument (10) inclut une tige de visée (12) destinée à être reliée à l'élément huméral (1 ) et présentant un trou à broche destiné à recevoir une broche (7) de positionnement dudit ensemble par rapport à l'os et au moins un trou de perçage d'un trou à vis (4).

Selon l'invention, l'instrument (10) comprend :
- au moins un premier tube rigide (13) formant ledit trou à broche, qui peut être engagé de manière ajustée dans un premier trou de réception (22) aménagé dans la tige de visée (12), et
- au moins un deuxième tube rigide (14) formant ledit trou de perçage, qui peut être engagé de manière ajustée dans un deuxième trou de réception (21) aménagé dans la tige de visée (12) alors que ledit premier tube rigide (13) est engagé dans ledit premier trou de réception (22).

## Description

La présente invention concerne un ensemble comprenant une prothèse d'épaule et un instrument de pose de cette prothèse d'épaule.

Une prothèse d'épaule comprend classiquement un élément huméral incluant une partie d'articulation et une tige effilée, cette tige effilée étant destinée à être engagée dans le canal médullaire de l'humérus, après résection adéquate, afin d'assurer l'ancrage de l'élément huméral à l'os.

La mise en place de la prothèse implique d'assurer un positionnement précis et une parfaite fixation de cet élément huméral par rapport à l'humérus, à défaut de quoi la reproduction de l'articulation naturelle par l'articulation prothétique ne serait pas parfaite. En particulier, l'élément huméral doit être parfaitement positionné et fixé dans le sens de son enfoncement par rapport à l'os, ainsi qu'en pivotement par rapport à celui-ci.

Une façon adaptée d'assurer ce positionnement précis et cette parfaite fixation consiste à prévoir au moins un trou dans la partie distale de ladite tige effilée et de mettre en place au moins une vis transversale au travers de la corticale osseuse et au travers de ce trou. Pour la mise en place de cette vis, il a été conçu un instrument comprenant une tige de visée destinée à être reliée, au niveau proximal, à la partie proximale de l'élément huméral, et présentant, au niveau distal, au moins un trou formant un guide de perçage de l'os, l'instrument comprenant également un organe de prise d'appui contre la partie proximale de l'os ainsi que des moyens de réglage de la position de cet organe par rapport à ladite tige de visée. Un tel instrument est décrit par la demande de brevet N° US 2003/149486. Pour la réalisation du perçage distal de l'os, l'instrument est relié à l'élément huméral (ou à un élément d'essai reproduisant la forme de cet élément huméral) puis la tige effilée de l'élément huméral ou de l'élément d'essai est introduite dans le canal médullaire de l'humérus, de telle sorte que ladite tige de visée s'étende le long de l'humérus ; les moyens de réglage sont ensuite actionnés de manière à faire porter ledit organe de prise d'appui contre la partie proximale de l'os puis de régler la position longitudinale de ladite tige de visée par rapport à l'os ; un ou plusieurs trous distaux de ladite tige de visée sont ensuite utilisés comme guides de perçage pour percer l'os. Ces trous, venant en regard des trous correspondants de la tige effilée, reçoivent les vis de fixation.

Cet instrument existant permet d'aménager un ou plusieurs trous distaux en un emplacement précis de l'os dans le sens longitudinal. Il ne donne cependant pas parfaitement satisfaction en pratique. En effet, l'aménagement du ou des trous distaux conserve un degré relatif d'imprécision, induisant un risque de non coïncidence parfaite d'un trou aménagé dans l'os avec le trou correspondant de la tige effilée de l'élément huméral. De plus, cet instrument a une structure relativement complexe, impliquant un coût de fabrication relativement important.

Il est par ailleurs connu de prévoir un ensemble comprenant:
- une prothèse d'épaule incluant un élément huméral qui comprend une tige effilée destinée à être engagée dans le canal médullaire de l'humérus, cette tige effilée comprenant au moins un trou à vis pour le verrouillage de cette tige par rapport à l'os au moyen de vis;
- un instrument incluant une tige de visée destinée à être reliée, au niveau proximal, à la partie proximale de l'élément huméral, et présentant un trou à broche destiné à recevoir en lui, de manière ajustée, une broche de positionnement dudit ensemble par rapport à l'os et au moins un trou de perçage d'un trou à vis.

Cet instrument ne permet pas de remédier aux inconvénients précités concernant le perçage de trous distaux.

La présente invention vise à remédier à ces inconvénients.

L'ensemble concerné comprend, de manière connue en soi,
- une prothèse d'épaule incluant un élément huméral qui comprend une tige effilée destinée à être engagée dans le canal médullaire de l'humérus, cette tige effilée comprenant au moins un trou à vis pour le verrouillage de cette tige par rapport à l'os au moyen de vis;
- un instrument incluant une tige de visée destinée à être reliée, au niveau proximal, à la partie proximale de l'élément huméral, et présentant un trou à broche destiné à recevoir une broche de positionnement dudit ensemble par rapport à l'os et au moins un trou de perçage d'un trou à vis.

Selon l'invention, l'instrument comprend :
- au moins un premier tube rigide formant ledit trou à broche, qui peut être engagé de manière ajustée dans un premier trou de réception aménagé dans la tige de visée, et
- au moins un deuxième tube rigide formant ledit trou de perçage, qui peut être engagé de manière ajustée dans un deuxième trou de réception aménagé dans la tige de visée alors que ledit premier tube rigide est engagé dans ledit premier trou de réception.

Ledit trou à broche n'est ainsi pas aménagé directement dans ladite tige de visée mais dans ledit premier tube rigide, qui est positionné de manière précise par rapport à la tige de visée grâce audit premier trou de réception et qui forme un guide pour la broche. L'emplacement d'insertion de la broche dans l'os est de cette façon déterminé de manière particulièrement précise, nonobstant une légère flexibilité longitudinale que présente inévitablement une telle broche. Cette broche est destinée à être engagée au travers du premier tube puis à être insérée dans l'os, permettant ainsi de fixer la position dudit ensemble par rapport à l'os. Une fois l'instrument positionné par rapport à l'os au moyen dudit premier tube et de la broche, ledit deuxième tube rigide est utilisé pour, alors que le premier tube et la broche sont en place, percer un ou plusieurs trous distaux dans l'os, ce deuxième tube constituant un guide de perçage permettant de déterminer de façon précise le ou les emplacements de perçage de l'os.

Il en résulte que l'instrument selon l'invention permet de déterminer précisément la position de l'instrument par rapport à l'os, sans nécessité d'un organe de prise d'appui et de moyens de réglage de la position de cet organe par rapport à la tige de visée, et qu'il permet d'aménager des trous à vis de manière très précise.

De préférence, la tige effilée de l'élément huméral comprend un trou à broche dans lequel peut être engagée, de manière ajustée, ladite broche de positionnement.

La broche de positionnement, en prenant ainsi appui tant au niveau de l'os qu'au niveau de la prothèse, permet non seulement de fixer la position dudit ensemble par rapport à l'os mais également de fixer précisément la position de la portion distale de la tige de visée par rapport à l'os. Une parfaite mise en correspondance du trou dudit deuxième tube avec un trou à vis de la prothèse est dès lors obtenue.

Ce trou à broche de la tige effilée traverse avantageusement cette tige effilée de part en part, de telle sorte que la broche de positionnement puisse traverser la portion distale de la tige effilée et que, le cas échéant, elle puisse ressortir de l'os du côté opposé à son côté d'introduction.

Avantageusement, le diamètre interne dudit deuxième tube rigide est tel qu'un dit premier tube rigide puisse être engagé de manière ajustée dans ce deuxième tube rigide.

Pour obtenir une précision encore renforcée quant à l'emplacement de perçage d'un trou osseux de réception d'une vis, ce premier tube rigide peut être mis en place dans ledit deuxième tube rigide de manière à permettre la mise en place d'une broche de guidage au niveau dudit emplacement, puis, après retrait de ce premier tube rigide, d'utiliser un foret canulé, engagé sur ladite broche, pour percer ledit trou osseux.

Avantageusement, la tige de visée, et ledit premier tube rigide et/ou ledit deuxième tube rigide, comprennent des moyens de maintien de ce ou ces tubes rigides par rapport à cette tige de visée, afin d'assurer le positionnement de ce ou ces tubes par rapport à cette tige.

Selon une possibilité, chaque tube rigide comprend une patte de préhension, facilitant sa manipulation. Dans ce cas, lesdits moyens de maintien d'un tube rigide par rapport à la tige de visée peuvent comprendre cette patte de préhension, d'une part, et au moins un logement aménagé dans la tige de visée, d'autre part, ce logement étant destiné à recevoir la patte de préhension de manière ajustée, avec éventuellement léger coincement.

L'instrument peut comprendre également une structure permettant, le cas échéant, la mise en place d'une vis proximale au niveau de la zone proximale de l'élément huméral, la tige effilée de cet élément huméral comprenant dans ce cas un trou correspondant pour le passage de cette vis proximale. Ladite structure comprend une partie de montage sur ladite tige de visée et comprend au moins un trou de perçage pour le perçage de l'os au niveau proximal.

L'instrument comprend avantageusement un troisième tube rigide formant ledit trou de perçage de l'os au niveau proximal, pouvant être engagé au travers d'un trou de réception que comprend à cet effet ladite structure.

De la même manière que précédemment, ce troisième tube rigide constitue un guide de perçage pour l'aménagement du trou à vis proximale, en un emplacement très précis.

L'instrument peut aussi comprendre un quatrième tube rigide dont le diamètre interne est tel que ce quatrième tube est apte à recevoir une broche en lui de manière ajustée, le diamètre interne dudit troisième tube rigide étant tel que ledit quatrième tube rigide puisse être engagé de manière ajustée dans ce troisième tube rigide.

Le quatrième tube, lorsqu'il est engagé dans le troisième tube permet la mise en place d'une broche au niveau proximal, laquelle permet le perçage du trou proximal dans l'os au moyen d'un foret canulé, donc avec une précision parfaite.

L'invention sera bien comprise, et d'autres caractéristiques et avantages de celle-ci apparaîtront, en référence au dessin schématique annexé, représentant, à titre d'exemple non limitatif, une forme de réalisation préférée de l'instrument et de la prothèse qu'elle concerne.

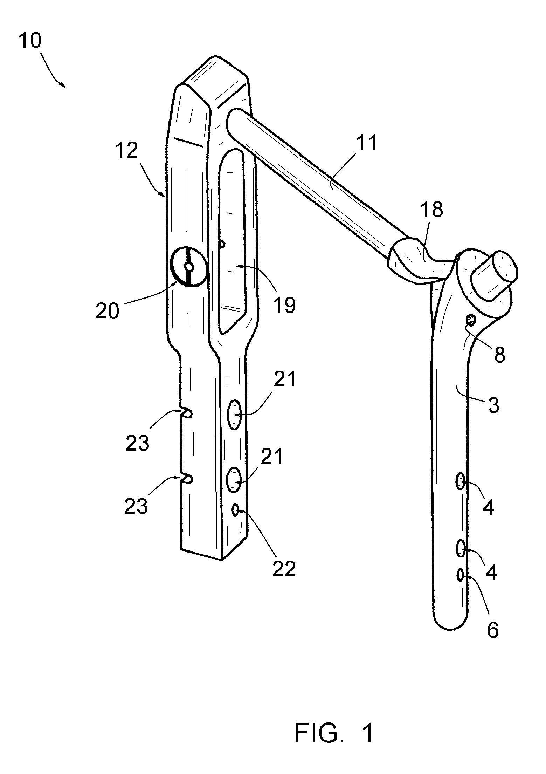

La figure 1 est une vue en perspective de la prothèse qu'elle concerne, reliée à une tige de visée que comprend l'instrument ;
la figure 2 est une vue en perspective de la prothèse mise en place dans un humérus, de ladite tige de visée et d'un premier tube rigide que comprend l'instrument ;
la figure 3 est une vue en perspective d'un deuxième tube rigide que comprend l'instrument, et
la figure 4 est une vue en perspective de la prothèse et de l'instrument, ce dernier étant équipé d'une structure de perçage proximal de l'os.

Les figures représentent un élément huméral 1 de prothèse d'épaule, et un instrument 10 de pose d'une prothèse d'épaule.

L'élément huméral 1 comprend, comme cela est classique, une tête d'articulation 2 et une tige effilée 3. Cette tige effilée 3 est destinée à être engagée dans le canal médullaire de l'humérus 100, après résection adéquate, afin d'assurer l'ancrage de l'élément huméral 1 à l'os.

La tige effilée 3 comprend deux trous à vis transversaux distaux 4, de réception de vis 5 d'ancrage à l'humérus 100, un trou à broche transversal traversant 6, de réception d'une broche 7, situé plus loin dans le sens distal que les trous 4, et un trou proximal 8 de réception d'une vis proximale (non représentée sur les figures) d'ancrage de la partie proximale de la tige effilée 3 à l'humérus 100.

La tige effilée 3 comprend également une zone proximale de montage (non visible sur les figures) pour sa liaison à une tige de liaison 11 que comprend l'instrument 10.

Pour le reste, la prothèse d'épaule est connue et ne fait pas en elle-même partie de l'invention, de sorte qu'elle ne sera pas décrite plus en détails.

L'instrument 10 comprend, outre la tige de liaison 11, une tige de visée 12, un premier tube rigide 13, un deuxième tube rigide 14 et une structure 15 de perçage proximal.

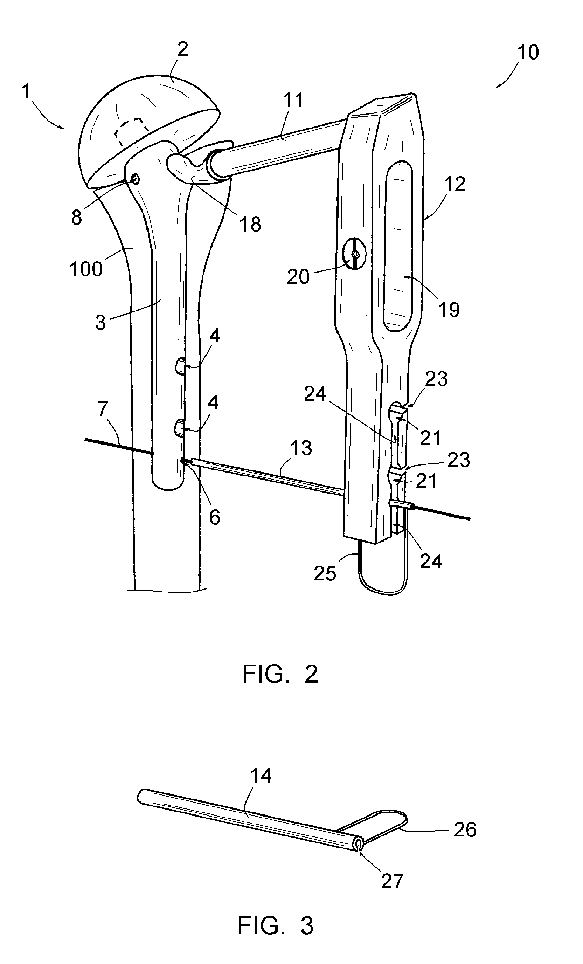

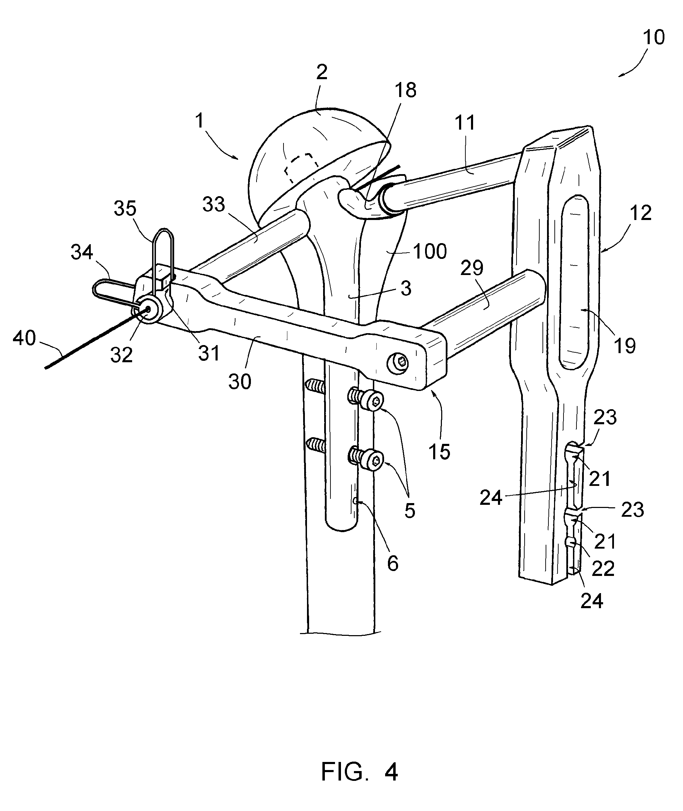

La tige de liaison 11 est reliée, par une extrémité, à la tige de visée 12 et comprend, à son autre extrémité, une extrémité de montage 18 complémentaire de la zone proximale de montage que comprend l'élément huméral 1. Comme cela apparaît sur les figures 2 et 4, cette tige de liaison 11 permet de relier l'élément huméral 1 à la tige de visée 12 de telle sorte que cette tige de visée 12 s'étende parallèlement à l'élément huméral 1, et à une distance de celui-ci telle que l'élément huméral 1 puisse être engagé dans le canal médullaire de l'humérus 100 et que la tige de visée 12 s'étende, dans cette position d'engagement de l'élément huméral 1, le long de l'humérus 100.

La tige de visée 12 présente une partie supérieure élargie, comprenant une lumière médiane 19, et une partie inférieure de largeur plus réduite. Sur chaque côté de la partie supérieure de la tige 12, sont aménagées deux zones de liaison 20 d'une tige 29 que comprend la structure 15, pour le montage de cette structure 15 sur la tige de visée 12, comme décrit plus loin. Dans la partie inférieure de la tige de visée 12, sont aménagés deux trous transversaux 21 de réception dudit deuxième tube rigide 14, un trou transversal 22, plus distal que les trous 21, de réception dudit premier tube rigide 13, deux encoches latérales 23 et deux rainures 24. Les deux encoches latérales 23 sont aménagées à la hauteur des trous 21, radialement par rapport à ceux-ci et perpendiculairement à la direction longitudinale de la tige de visée 12, et débouchent dans la face latérale de cette tige 12. Les deux rainures 24 relient les trous 21 entre eux et le trou 21 distal à l'extrémité distale de la tige de visée 12, en passant par le trou 22 ; elles débouchent dans la face antérieure de la tige de visée 12, c'est-à-dire la face opposée à celle destinée à être tournée vers l'élément huméral 1, sans déboucher dans la face postérieure de cette tige 12.

Le premier tube rigide 13 présente un diamètre interne tel qu'il peut recevoir, avec coulissement et de manière ajustée, la broche 7, et un diamètre externe tel qu'il peut être reçu, avec coulissement et de manière ajustée, dans le trou distal 22. Ce tube 13 comprend une patte de préhension 25, facilitant sa manipulation, sous forme d'un fil recourbé en "U".

Comme le montre la figure 2, lorsque le tube 13 est destiné à être engagé dans le trou 22, la patte 25 prend place dans la rainure 24, éventuellement avec un léger coincement, ce qui permet de caler et éventuellement d'immobiliser le tube 13 par rapport à la tige de visée 12.

Il apparaît sur la figure 3 que le tube rigide 14 présente une structure identique à celle du tube 13, avec une patte de préhension 26 sous forme d'un fil recourbé en "U", à l'exception du fait qu'il comprend une encoche 27 débouchant dans son extrémité antérieure, propre à recevoir la patte 25 du tube 13. Le tube 14 a un diamètre externe tel qu'il peut être reçu, avec coulissement et de manière ajustée, dans l'un ou l'autre des trous distaux 21, la patte 26 prenant place dans l'une ou l'autre des rainures 24 ou des encoches 23. Ce tube 14 a un diamètre interne tel qu'il peut recevoir le tube 13, avec coulissement et de manière ajusté.

La structure 15 de perçage proximal comprend, outre la tige de liaison 29, une barrette 30 pouvant être reliée rigidement à cette tige de liaison 29 de telle sorte que cette barrette 30 s'étende sensiblement orthogonalement à la tige de visée 12. La barrette 30 comprend, du côté opposé à la tige 12, un trou traversant au niveau duquel sont aménagées deux encoches latérales 31, dont une débouche dans la face latérale de la barrette 30 et l'autre dans la face d'extrémité de cette barrette 30.

Il apparaît sur la figure 4 que le trou traversant de la barrette 30 est destiné à recevoir un troisième tube rigide 32 et un quatrième tube rigide 33, de structures identiques à celles respectivement des tubes 13 et 14. Le tube 32 a un diamètre interne tel qu'il peut recevoir, avec coulissement et de manière ajustée, une broche 40, et un diamètre externe tel qu'il peut être engagé, avec coulissement et de manière ajustée, dans le tube 33; la patte 34 que comprend ce tube 32 peut être engagée, avec éventuellement un léger coincement, dans l'encoche 31 débouchant dans l'extrémité de la barrette 30, afin de caler et de maintenir ce tube 32 par rapport à la barrette 30. Le tube 33 a un diamètre interne tel qu'il peut recevoir, avec coulissement et de manière ajustée, le tube 32, et un diamètre externe tel qu'il peut être engagé, avec coulissement et de manière ajustée, dans ledit trou traversant de la barrette 30 ; de la même manière que précédemment, la patte 36 que comprend ce tube 33 peut être engagée, avec léger coincement, dans l'encoche 31 débouchant dans la face latérale de la barrette 30, afin de caler et de maintenir ce tube 33 par rapport à la barrette 30.

En pratique, après engagement de l'élément huméral 1 dans l'humérus 100, le tube 13 est engagé au travers du trou 22 jusqu'à engagement et coincement de sa patte 25 dans la rainure 24, puis la broche 7 est engagée dans ce tube puis au travers de l'os 100 et du trou 6.

La mise en place de cette broche 7 permet de parfaitement immobiliser la tige de visée 12 par rapport à l'os 100.

Le tube rigide 14 est alors engagé dans l'un des trous 21 jusqu'à coincement de sa patte 26 dans la rainure 24 ou l'encoche 23 correspondante, en vue de percer l'os 100 en regard d'un trou 4. Ce tube 14 peut être utilisé seul pour opérer ce perçage au moyen d'un foret non canulé; alternativement, le tube 13 est engagé dans le tube 14, la patte 25 prenant place dans l'encoche 27 et dans la rainure 24, et la patte 26 prenant place dans l'encoche 23 ; le tube 13 est utilisé pour la mise en place d'une broche puis, après retrait du tube 13, un foret canulé est utilisé pour percer l'os en regard du trou 4 correspondant.

Les opérations ci-dessus sont répétées pour le perçage de l'autre trou osseux, en regard de l'autre trou 4.

Lorsqu'il y a lieu de réaliser un perçage proximal permettant la mise en place d'une vis au travers du trou 8, la structure 15 est montée sur la tige de visée 12, et soit le tube rigide 33 est utilisé seul pour opérer ce perçage proximal, soit les tubes rigides 32 et 33 sont utilisés conjointement, de la même manière qu'indiqué ci-dessus pour les tubes 13 et 14, afin d'opérer ce perçage au moyen d'une broche et d'un foret canulé.

Comme cela apparaît de ce qui précède, l'invention fournit un ensemble instrument - prothèse d'épaule présentant, par rapport aux instruments homologues de la technique antérieure, les avantages déterminants de permettre de fixer précisément la position de la portion distale de la tige de visée 12 par rapport à l'os 100, de permettre d'aménager un ou plusieurs trous distaux selon une position parfaitement précise, et d'avoir en outre une structure relativement simple.

L'invention a été décrite ci-dessus en référence à une forme de réalisation donnée à titre de pur exemple Il va de soi qu'elle s'étend à toutes les formes de réalisations couvertes par les revendications ci-annexées.

## Revendications

1. Ensemble comprenant une prothèse d'épaule et un instrument (10) de pose de cette prothèse d'épaule (1), la prothèse d'épaule incluant un élément huméral (1) qui comprend une tige effilée (3) destinée à être engagée dans le canal médullaire de l'humérus, cette tige effilée (3) comprenant au moins un trou à vis (4) pour le verrouillage de cette tige (3) par rapport à l'os au moyen de vis (5), et l'instrument (10) incluant une tige de visée (12) destinée à être reliée, au niveau proximal, à la partie proximale de l'élément huméral (1), et présentant un trou à broche destiné à recevoir une broche (7) de positionnement dudit ensemble par rapport à l'os et au moins un trou de perçage d'un trou à vis (4) ;
**caractérisé en ce que** l'instrument (10) comprend :
- au moins un premier tube rigide (13) formant ledit trou à broche, qui peut être engagé de manière ajustée dans un premier trou de réception (22) aménagé dans la tige de visée (12), et
- au moins un deuxième tube rigide (14) formant ledit trou de perçage, qui peut être engagé de manière ajustée dans un deuxième trou de réception (21) aménagé dans la tige de visée (12) alors que ledit premier tube rigide (13) est engagé dans ledit premier trou de réception (22).

2. Ensemble selon la revendication 1, **caractérisé en ce que** la tige effilée (3) de l'élément huméral (1) comprend un trou à broche (6) dans lequel peut être engagée, de manière ajustée, ladite broche de positionnement (7).

3. Ensemble selon la revendication 2, **caractérisé en ce que** le trou à broche (6) de la tige effilée (3) traverse cette tige effilée (3) de part en part, de telle sorte que la broche de positionnement (7) puisse traverser la portion distale de la tige effilée (3).

4. Ensemble selon l'une des revendications 1 à 3, **caractérisé en ce que** le diamètre interne dudit deuxième tube rigide (14) est tel qu'un dit premier tube rigide (13) puisse être engagé de manière ajustée dans ce deuxième tube rigide (14).

5. Ensemble selon l'une des revendications 1 à 4, **caractérisé en ce que** la tige de visée (12), ledit premier tube rigide (13) et/ou ledit deuxième tube rigide (14), comprennent des moyens (25, 26, 23, 24) de maintien de ou ces tubes rigides (13, 14) par rapport à cette tige de visée (12), afin d'assurer le positionnement de ce ou ces tubes (13, 14) par rapport à cette tige (12).

6. Ensemble selon l'une des revendications 1 à 5, **caractérisé en ce que** chaque tube rigide (13, 14) comprend une patte de préhension (25, 26), facilitant sa manipulation.

7. Ensemble selon la revendication 5 et la revendication 6, **caractérisé en ce que** lesdits moyens de maintien d'un tube rigide (13, 14) par rapport à la tige de visée (12) comprennent ladite patte de préhension (25, 26), d'une part, et au moins un logement (23, 24) aménagé dans la tige de visée (12), d'autre part, ce logement (23, 24) étant destiné à recevoir la patte de préhension (25, 26) de manière ajustée, avec éventuellement léger coincement.

8. Ensemble selon l'une des revendications 1 à 7, **caractérisé en ce que** :
- la tige effilée (3) de l'élément huméral (1) comprend un trou proximal (8) pour le passage d'une vis proximale d'ancrage à l'humérus (100); et
- l'instrument (10) comprend une structure (15) permettant la mise en place d'une vis proximale au niveau de la zone proximale de l'élément huméral (1), cette structure (15) comprenant une partie (11) de montage sur ladite tige de visée (12) et au moins un trou pour le perçage de l'os (100) au niveau proximal.

9. Ensemble selon la revendication 8, **caractérisé en ce que** l'instrument (10) comprend un troisième tube rigide (33) formant ledit trou pour le perçage de l'os (100) au niveau proximal, pouvant être engagé au travers d'un trou que comprend ladite structure (15).

10. Ensemble selon la revendication 9, **caractérisé en ce que** l'instrument (10) comprend un quatrième tube rigide (32) dont le diamètre interne est tel que ce tube (32) est apte à recevoir une broche (40) en lui de manière ajustée, le diamètre interne dudit troisième tube rigide (33) étant tel que ledit quatrième tube rigide (32) puisse être engagé de manière ajustée dans ce troisième tube rigide (33).
